# EUROPEAN PATENT APPLICATION

(11) **EP 4 223 280 A1**
(43) Date of publication of application: **09.08.2023**
(21) Application number: 22154688.0
(22) Date of filing: 02.02.2022
(51) Int. Cl.: A61K 9/00, A61K 9/06, A61K 47/10, A61K 31/00, A61P 1/00

(54) **DICLOFENAC FORMULATIONS**

(71) Applicant: GSK Consumer Healthcare SARL, 1197 Prangins (CH)
(72) Inventor: CHEIGNON, Clotilde, Nyon (CH); LANDER, Jean Philippe, Nyon (CH); PRADAL, Julie, Nyon (CH)
(74) Representative: Haleon Patent Department

(57) **Abstract**

A monophasic, hydroalcoholic gel for topical drug delivery, comprising diclofenac epolamine or diclofenac diethylammonium, in a concentration of about 0.5% to about 1.5% w/w, a C₂-C₄-monoalcohol, in a concentration of about 12% to about 15% w/w, a C₂-C₄-polyalcohol, in a concentration of about 2.5% to about 7.5% w/w, a gelling agent, optionally, pH adjusting agent, and water, wherein the pH of the total composition is from about 6.5 to about 8.5. The compositions are for use of topical treatment of pain.

## Description

### FIELD OF THE INVENTION

This disclosure relates to formulations for the topical delivery of diclofenac. Specifically, it relates to gel formulations comprising about 0.5% to 1.5% (w/w) of diclofenac diethylammonium salt.

### BACKGROUND TO THE INVENTION

Diclofenac is a non-steroidal anit-inflammatory drug (NSAID) of the acetic acid class widely used in topical or transdermal products. Emulsion gels, also called emulgels are oil-in-water emulsions with a jellified aqueous phase. Emulgel formulations have been successful formats for diclofenac topical delivery. Despite emulgels being successful in the market, recent patient reports suggest that some users are dissatisfied with some of their attributes. The present inventors have overcome those disadvantages, and the present disclosure provides a topical diclofenac product that has the similar advantages of the exisiting products, but additionally has (sensory) attributes that satisfy the evolving consumer preferences.

### SUMMARY OF THE INVENTION

There is provided a monophasic, hydroalcoholic gel for topical drug delivery, comprising
a) diclofenac epolamine or diclofenac diethylammonium, in a concentration of about 0.5% to about 1.5% w/w,
b) a C₂-C₄-monoalcohol, in a concentration of about 12% to about 15% w/w,
c) a C₂-C₄-polyalcohol, in a concentration of about 2.5% to about 7.5% w/w,
d) a gelling agent,
e) optionally, pH adjusting agent,
f) water,
   wherein the pH of the total composition is from about 6.5 to about 8.5.

In one embodiment, component b) is isopropanol.

In one embodiment, component c) is propylene glycol.

In one embodiment, components b) and c) together are present in a concentration of about 15% to about 25% w/w.

In one embodiment, components b) and c) together are present in a concentration of about 15% to about 20% w/w.

In one embodiment, the ratio of components b) to c) is from about 1:1 to about 5:2.

In one embodiment, component b) is present in a concentration of about 12% to about 13% w/w.

In one embodiment, component c) is present in a concentration of about 4% to about 6% w/w.

In one embodiment, component d) is a Carbomer gelling agent.

In one embodiment, component e) is a basic agent.

In one embodiment, component a) is diclofenac diethylammonium.

In one embodiment, component f) is present in a concentration of more than about 70% w/w.

In one embodiment, component d) is present in a concentration of about 0.7% to about 1.5% w/w.

In one embodiment, the gel is substantially free from permeation enhancers.

In one embodiment, the gel is substantially free from emulsifying agents.

In one embodiment, the gel is substantially free from humectants.

In one embodiment, the gel does not contain a lipophilic phase.

In one embodiment, the viscosity of the gel is from about 1Pa.S to about 5Pa.S.

In one embodiment, the gel has a drying time on skin of less than 120 seconds.

In one embodiment, the gel has cumulative permeation in an in vitro skin permeation test, that enables to bridge the literature data of Voltaren 1.16% Diclofenac Diethylammonium emulgel, in view of well-established use registration, according to Annex I of Directive 2001/83/EC.

In one embodiment, the gel is transparent.

In one embodiment, the gel consists essentially of:
a) diclofenac diethylammonium, in a concentration of about 1% to about 1.2% w/w,
b) isopropanol, in a concentration of about 12% to about 13% w/w,
c) propylene glycol, in a concentration of about 4% to about 6% w/w,
d) Carbomer, in a concentration of about 0.7% to about 1.5% w/w,
e) diethylamine, in a concentration of about 0.7% to about 1.3% w/w,
f) optionally, one or more of perfumes, pH adjusting agents, gelling agents, emollients, humectants, preservatives, chelating agents, antioxidants and/or colorants.

In one embodiment, the gel is provided in an applicator pack for direct application to the skin.

In one embodiment, the gel is for use in a method of providing a cooling sensation on mammal skin on an application site.

In one embodiment, the gel is for use in the treatment of a human suffering from joint pain, osteoarthritis pain, back pain, knee pain, ankle pain, neck pain, muscle pain, sprains, and/or inflammation.

In one embodiment, the gel is applied to the skin of a site of application in a dose of 2grams on upper extremities or 4 grams on lower extremities, the gel is rubbed or massaged into the skin until the application site is dry, and the application is repeated 3 to 4 times in 24 hours, until treatment is ceased.

### DESCRIPTION OF DRAWINGS/FIGURES

FIG. 1: Representative photograph of a composition of this disclosure in comparison to Voltaren 1.16% Emulgel
FIG. 2: Representative photograph of a composition of this disclosure on a typical dosing card for topical products.
FIG. 3: Representative photograph of Voltaren 1.16% Emulgel on a typical dosing card for topical products.

### DETAILED DESCRIPTION OF THE INVENTION

An example of a topical diclofenac formulation is Voltaren Arthritis Pain (diclofenac sodium topical gel, 1% (NSAID)- arthritis pain reliever) which comprises 1 % w/w diclofenac sodium and is approved in the USA as an over the counter (OTC) drug since 2020. Another topical diclofenac formulation comprises 1.16% w/w diclofenac diethylammonium salt (equivalent to 1% of the diclofenac sodium salt), sold as Voltaren or Voltarol 1.16% Emulgel. Voltaren 1.16% Emulgel and has been marketed in Europe since 1985. Voltaren Arthritis Pain and Voltaren 1.16% Emulgel are emulgels. The emulgel format has been described in combination with the active ingredient (API) diclofenac e.g. in US4917886.

According to US4917886, the described emulgel formulations provide a readier solubility of the active ingredient, and an associated higher effective ingredient concentration compared to conventional topical formulations such as gels or creams. Another advantageous property of the emulgels is described to be the presence of a lipid phase which provides fat-restoring properties. The combination of a lipid phase with a gelled aqueous phase enables the formulation to be massaged in whilst, at the same time, the direct absorption into the skin is experienced as a pleasant property.

Existing Voltaren 1% or 1.16% emulgel products are successful commercial products. However, due to development of consumer preference over time, some attributes of these commercial formulations do not appeal to some consumers.

Recent patient reports and consumer preference studies suggest that some patients find the products too greasy. Some users report that the products leaves sticky traces on the application site. Some users report that they find the application "messy", because they have to apply the product, which contains fatty ingredients, by hand, and then wash their hands to remove the fatty components from their hands.

Additionally, colored or opaque products are harder to dose with conventional dosing cards used for topical products, as they cover the scale or measurement area.

Additionally some consumers prefer transparent or clear gels. These are perceived as modern, clean, fresh, and natural. Voltaren Arthritis pain and Voltaren 1.16% Emulgel however are opaque to white.

Additionally, some consumers expressed a preference for a topical product for the treatment of their pain, that, in addition to efficient drug delivery, provides a cooling sensation on the application site. This is perceived as a marker for rapid onset of pain relief or immediate action of the formulation.

The present inventors therefore were seeking to improve those attributes of the formulation, whilst keeping the modifications compared to the current Voltaren 1% or 1.16% emulgel products to a minimum. This approach was taken to maintain the advantageous properties of Voltaren 1% or 1.16% emulgel products, which patients are familiar with and appreciate. It was also taken in a view of patient safety: By limiting the modifications vis-à-vis exisiting, marketed products with an excellent safety profile, the risk of adverse events caused by the new composition is reduced. Moreover, registration of a new pharmaceutical product with regluatory authorities can be facilitated for products that are similar or comparable to already approved products. Additionally, this approach allows to leverage exisiting supply chains and manufacturing capabilities.

It was another objective of the present invention to provide a product which could be registered under the "well established use" criteria, relying on Voltaren 1.16% Emulgel as comparator product.

The compositions disclosed herein are compositions for topical drug delivery. This means, the compositions comprise an active pharmaceutical ingredient (API) and are intended to be applied on skin of a patient in need of treatment with that API. The compositions are effective vehicles for the API to permeate skin. This is necessary for the API to reach a site of action. The compositions disclosed herein therefore allow for the API to permeate through skin and to a site of action, e.g. a joint. Effective delivery of the API through the skin can be measured in pre-clinical experiments, which is further described herein.

There is provided a monophasic, hydroalcoholic gel for topical drug delivery. Monophasic is to be understood as a composition that comprises a single phase, as opposed to multi-phase compositions such as emulsions, creams, suspensions or the like. In the present case, the single phase is a continous phase comprising water. A hydroalcoholic gel means that the gel comprises a combination of water as the continous phase and one or more water miscible alcohol(s), optionally comprising additional water-miscible excipients. This is a completely different delivery format compared to, for example, the multi-phase emulgels previously described herein, or creams and ointments, which are also conventionally used for topical delivery. Those multi-phase systems comprise a hydrophilic (emulgels) or lipophilic (creams) continous phase and a disperse phase, which is dispersed in the continous phase. The disperse phase in these multi-phase systems refracts light. This makes multi-phase systems appear opaque or colored, normally opaque white. Monophasic compositions disclosed herein do not contain a disperse phase which refracts light. Thus, compositions disclosed herein are transparent.

The compositions disclosed herein comprise an API that is a pharmaceutically acceptable salt of diclofenac. Preferably, salts of diclofenac with organic bases or ammonia are used. For example, the ammonium salt, alkylamine salt dimethylamine, diethylamine, trimethylamine and a cyclic amine salt such as epolamine (hydroxyethylpyrrolidine salt) can be used. Preferably, the compositions disclosed herein comprise diclofenac diethylammonium salt.

In some embodiments, compositions described herein are substantially free of additional APIs. This means, that no API second or further API is present in the composition in those embodiments. In a preferred embodiment, diclofenac is the only API in the composition. For example, the compositions do not contain an additional NSAID, such as ibuprofen.

*Substantially free of,* in the present disclosure, is to be understood as meaning that no additional ingredients are added to the formulation that have a certain functionality. For example, *substantially free of permeation enhancers,* means that no permeation enhancer is added to the compositions. This however does not exclude the additional ingredients explicitly listed in this disclosure. Also, in pharmaceutical compositions, many ingredients perform more than one function. It thus may be that an additional ingredient listed herein, in addition to its functionality described herein, excerts certain other functionalities. For example, a solvent, which is described as a additional component in some embodiments of compositions described herein, may also act as a permeation enhancer. In this example, *substantially free of permeation enhancers* would not exclude the presence of this solvent in the compositions of this disclosure. Additionally, compositions may contain insignificant or trace amounts of certain compounds. For example this can occur due to impurities or degradation products. Those small or trace amounts are also not encompassed by the term, and such compositions would still be considered to be *substantially free* of that compound.

The compositions of this disclosure can comprise a diclofenac salt in a concentration of about 0.5% w/w to about 1.5% w/w of diclofenac sodium equivalent.

The compositions of this disclosure can comprise diclofenac diethylammonium in a concentration of about 0.58% w/w to about 1.75% w/w. This is equivalent to about 0.5% w/w to about 1.5% w/w of diclofenac sodium.

Preferably, the compositions comprise diclofenac diethylammonium in a concentration of about 1% to about 1.2% w/w. In some embodiments, the compositions can comprise diclofenac diethylammonium in a concentration of about 1% w/w, 1.01% w/w, 1.02% w/w, 1.03% w.w, 1.04% w/w, 1.05% w/w, 1.06% w/w, 1.07% w/w, 1.08% w/w, 1.09% w/w, 1.10% w/w, 1.11% w/w, 1.12% w/w, 1.13% w/w, 1.14% w/w, 1.15% w/w, 1.16% w/w, 1.17% w/w, 1.18% w/w, 1.19% w/w, or 1.2% w/w. Especially preferred is diclofenac diethylammonium in a concentration of about 1.16% w/w. In instances where a different pharmaceutically acceptable salt of diclofenac is used, one of ordinary skill in the art would understand that the corresponding equivalent amounts are used.

In higher concentrations, the API can sometimes re-crystallize in the final formulation, such that crystals of API are present in the product. Non-solubilized API can not permeate the skin and is therefore lost. Lost API will stay on the skin and ultimately be washed off and end in waste water. Additionally, lost API raises the cost of a product without supporting efficacy. The presence of unsolubilized API also hampers dosage uniformity, which is not acceptable in a view of patient safety. Therefore, there is a need to prevent API loss from non-solubilized API, and to maintain fully solubilized API, inlcuding during storage. This is discussed in more detail in a separate paragraph on stability.

The compositions disclosed herein comprise a C₂-C₄-monoalcohol. The C₂-C₄-monoalcohol can be selected from methanol, ethanol, propanol, iso-propanol and butanol, and mixtures thereof. In some preferred embodiments, the compositions comprise ethanol and isopropanol and mixtures thereof. In some embodiments, the compositions comprise isopropanol.

The C₂-C₄-monoalcohol can act as a solubilizer of the API. Moreover, it has a low evaporation temperature. Upon application to the skin, the C₂-C₄-monoalcohol evaporates, which can be perceived as a cooling sensation on the skin. This provides a perception of immediate action, as desired by consumers. These C₂-C₄-monoalcohols are colourless and transparent, which is beneficial for the compounding of clear or transparent gels of the present disclosure. They are therefore suitable for the formulation of a colourless, transparent topical gel, which is preferred by consumers. The disclosed C₂-C₄-monoalcohols also impart an antibacterial or anti-germ activity to the formulation, improving the microbial stability.

In some embodiments, isopropanol is used as the sole C₂-C₄-monoalcohol. It has been found that isopropanol is sufficient to solubilise the API, and further provides an immediate cooling sensation on the skin of a user. Moreover, it has been discovered that isopropanol can be used as the sole C₂-C₄-monoalcohol in compositions herein without another C₂-C₄-monoalcohol that evaporates more readily, such as ethanol. This is preferred because the number of different ingredients can be kept low which facilitates manufacturing, reduces production cost and simplifies supply chain. Moreover, ethanol is regulated in many states for tax or ethical and religious reasons.

The C₂-C₄-monoalcohol can be present in the compositions in concentrations of about 12% w/w to about 15% w/w. In a preferred embodiment, the C₂-C₄-monoalcohol can be present in a concentration of about 12% w/w to about 13% w/w. In one embodiment, the C₂-C₄-monoalcohol is present in a concentration of about 12.5% w/w. Alternatively, the C₂-C₄-monoalcohol can be present in the compositions in concentrations of about 12% w/w, about 13% w/w, about 13.5% w/w, about 14% w/w, about 14.5% w/w, or about 15% w/w.

These concentrations of C₂-C₄-monoalcohol aid to solubilise the API. Additionally, these concentrations of C₂-C₄-monoalcohol produce an adequate cooling sensation upon application. Lower concentrations of C₂-C₄-monoalcohol can in some instances produce less cooling sensation, for example, the cooling sensation may to too faint or the cooling sensation may not last long enough.

If the concentration of C₂-C₄-monoalcohol exceeds the ranges cited above, it can occur that, upon application of the composition to the skin, the C₂-C₄-monoalcohol component evaporates. The remainder of the composition on the skin can then be insufficient to solubilize the API. The API thus can crystallize on the skin, leaving a film or brittle precipitate, on the skin. This is not desirable due to the lost API considerations explained above. It is also not desirable because it would result in an unpleasant user experience. Moreover, higher concentrations of C₂-C₄-monoalcohol may require a product to be labelled, stored, and handled under high scrutiny, as it may be characterized to be a hazardous material under certain regulations. This is not desirable as it complicates manufacture and supply chain and may deter consumers from using the final product. Alcohols also have a certain odour that may be deterring some consumers. Especially in monophasic, hydroalcoholic compositions, it is challenging to incorporate certain perfumes or odour masking agents, as those are often lipophilic, which can lead to phase separation, creaming, droplet formation, cloudy or hazy appearance of the product or the like. Therefore, it is beneficial to keep the concentration of C₂-C₄-monoalcohol to the minimum necessary, to avoid the need for addition of (more) perfumes or odour masking agents. Additionally, C₂-C₄-monoalcohols have certain lipolytic properties that can have a negative effect on the natural fat and ceramide components of skin, particularly human skin. To prevent dehydrating and irritating effects to the skin, especially in the absence of fat-restoring oil phase, formulators may try to keep the concentration of C₂-C₄-monoalcohols in the topical compositions as low as possible. C₂-C₄-monoalcohols can also have an influence on the permeation properties of a formulation. However, any such effect on permeation is also dependent on other components and the overall composition of the product. The present inventors aimed at formulating a composition with permeation characteristics similar to those of the existing Voltaren 1% or 1.16% Emulgel products. By that, the posology, i.e. the dosage amounts and frequencies of the existing products can be maintained. This is explained in more detail in a separate section below.

In one embodiment, the gel of the present disclosure comprises isopropanol in a concentration of about 12% w/w to about 15% w/w, preferably about 12% w/w to about 13% w/w, and no other additional C₂-C₄-monoalcohol, as previously disclosed. Isopropanol in these ranges is particularly advantageous as it is sufficient to provide solubilisation and cooling whilst avoiding the negative effects that may occur in formulations which contain other concentrations of C₂-C₄-monoalcohol. Particularly, the present inventors have found that compositions comprising isopropanol in the recited ranges have permeation characteristics similar to those of the existing Voltaren 1% or 1.16% Emulgel products.

The compositions disclosed herein comprise a C₂-C₄-polyalcohol. The C₂-C₄-polyalcohol typically is a glycol. The C₂-C₄-polyalcohol can be selected from ethylene glycol, propylene glycol and 1,3-butylene glycol, and mixtures thereof. Propylene glycol is preferred. In some embodiments, propylene glycol is used as the sole C₂-C₄-polyalcohol. The C₂-C₄-polyalcohol acts as a solubilizer of the API. In contrast to the C₂-C₄-monoalcohol, the C₂-C₄-polyalcohol does not evaporate readily when the composition is applied to the skin. The of C₂-C₄-polyalcohol therefore remains on the skin even after evaporation of the C₂-C₄-monoalcohol, and ensures that the API stays solubilized. C₂-C₄-polyalcohols can also have humecting, water retaining properties. This can provide a pleasant skin feeling, such as a feeling of soft, supple, or nurtured skin. C₂-C₄-polyalcohols can also have an influence on the permeation properties of a formulation. However, this effect on permeation can also depend on other components and the overall composition of the product. As discussed above, the present inventors aimed at formulating a composition with permeation characteristics similar to those of the existing Voltaren 1% or 1.16% emulgel products, which is explained in more detail in a separate section below.

The C₂-C₄-polyalcohol can be present in the compositions in a concentration of about 2.5% w/w to about 7.5% w/w. Preferably, the C₂-C₄-polyalcohol can be present in the compositions in a concentration of about 4% w/w to 6% w/w. In one embodiment, the C₂-C₄-polyalcohol is present in a concentration of about 5% w/w. Alternatively, the C₂-C₄-polyalcohol can be present in a concentration of about 2.5% w/w, about 3% w/w, about 3.5% w/w, about 4% w/w, about 4.5% w/w, about 5% w/w, about 5.5% w/w, about 6% w/w, about 6.5% w/w, about 7% w/w, or about 7.5% w/w.

It was found that the disclosed concentrations of C₂-C₄-polyalcohol solubilize the API and keep the API in solubilisation after application of the composition to the skin, when the C₂-C₄-monoalcohol evaporates. The disclosed concentrations were also found to provide safe compositions that do not cause skin irritation, which can sometimes be caused by compositions comprising excess concentrations of C₂-C₄-polyalcohol. Additionally, the disclosed concentrations of C₂-C₄-polyalcohol do not immoderately prolong the time that the compositions needs to be rubbed or let dry on an application area, until a user perceives the application area as dry. The disclosed concentrations of C₂-C₄-polyalcohol yield compositions with a tailored drying time. Additionally, the disclosed concentrations of C₂-C₄-polyalcohol yield compositions with low perception of stickiness on the skin. Compositions comprising the disclosed concentrations of C₂-C₄-polyalcohol can be formulated as clear and transparent gels, without the hazy or cloudy appearance that compositions comprising C₂-C₄-polyalcohols in higher concentrations can have.

In one embodiment, the compositions of the present disclosure comprise propylene glycol in a concentration of about 2.5% w/w to about 7.5% w/w, preferably about 4% w/w to about 6% w/w, and no other additional C₂-C₄-polyalcohol. Propylene glycol in a concentration of about 2.5% w/w to about 7.5% w/w, preferably about 4% w/w to about 6% w/w, is sufficient to achieve good solubilisation of the API in the concentration ranges described above. At the same time, these concentrations of propylene glycol are low enough to prevent the negative effects described above. Importantly, these concentrations of about 2.5% w/w to about 7.5% w/w, preferably about 4% w/w to 6% w/w, yield clear, transparent gels.

In an embodiment, the compositions comprise a combination of isopropanol and propylene glycol as the C₂-C₄-monoalcohol and the C₂-C₄-polyalcohol.

The ratio of C₂-C₄-monoalcohol to C₂-C₄-polyalcohol can be from about 1:1 to about 5:2. Preferred is a ratio of C₂-C₄-monoalcohol to C₂-C₄-polyalcohol of 5:2.

The total combined concentration of C₂-C₄-monoalcohol and C₂-C₄-polyalcohol more than about 15% w/w in some embodiments. The total combined concentration of C₂-C₄-monoalcohol and C₂-C₄-polyalcohol preferably is less than about 25% w/w. Expressed in ranges, the total combined concentration of C₂-C₄-monoalcohol and C₂-C₄-polyalcohol can be about 15% w/w to about 25% w/w, preferably about 15% w/w to about 20% w/w. In some cases, the total combined concentration of C₂-C₄-monoalcohol and C₂-C₄-polyalcohol is about 15% w/w, about 15.5% w/w, about 16% w/w, about 16.5% w/w, about 17% w/w, about 17.5% w/w, about 18% w/w, about 18.5% w/w, about 19% w/w, about 19.5% w/w, about 20% w/w, about 20.5% w/w, about 21% w/w, about 21.5% w/w, about 22% w/w, about 22.5% w/w, about 23% w/w, about 23.5% w/w, about 24% w/w, about 24.5% w/w, or about 25% w/w. In one embodiment, the total combined concentration of C₂-C₄-monoalcohol and C₂-C₄-polyalcohol is about 17.5% w/w.

The compositions of the disclosure also comprise a gelling agent. In some embodiments, the compositions comprise a carbomer gelling agent. Carbomer gelling agents yield very clear, transparent gels in many cases. They are also preferred for their spreading characteristics that are preferred by many consumers over those of other gelling agents, such as hydroxypropyl methyl cellulose. For example, Carbomer 974P or Carbomer 980 can be used. Carbomers are unlikely to form a film or flakes on the skin after application of the compositions onto the skin.

The carbomer gelling agent can be present in concentrations sufficient to raise the viscosity of the composition. In some embodiments the carbomer gelling agent can be present in concentrations of about 0.7% w/w to about 1.5% w/w. In one embodiment, the compositions of the present disclosure comprise carbomer in a concentration of about 1.2 % w/w. In some embodiments the carbomer gelling agent can be present in concentrations of about 0.8% w/w, about 0.9% w/w, about 1% w/w, about 1.1% w/w about 1.2% w/w, about 1.3%, about 1.4% w/w or about 1.5% w/w.

It can be advantageous that the viscosity is high enough to allow the composition to be dispensed from a tube and that the composition is not too runny or drippy. Preferably, the viscosity of the final composition is from about 1Pa.S to about 5Pa.S, more preferred from about 1.8Pa.S to about 4Pa.S at 20°C. In some instances, the viscosity of the final composition is about 1Pa.S, about 1.2Pa.S, about 1.4Pa.S, about 1.6Pa.S, about 1.8Pa.S, about 2Pa.S, about 2.2Pa.S, about 2.4Pa.S, about 2.6Pa.S, about 2.8Pa.S, about 3Pa.S, about 3.2Pa.S, about 3.4Pa.S, about 3.6Pa.S, about 3.8Pa.S, about 4 Pa.S, about 4.2Pa.S, about 4.4Pa.S, about 4.6Pa.S, about 4.8Pa.S, or about 5Pa.S at 20°C. In one embodiment, the viscosity of the final composition is from about 20 about 100 scale units, more preferred from about 40 to 80 scale units, measured at 25°C. In some embodiments, the viscosity of the final composition is about 20 about 100 scale units, about 30 scale units, about 40 scale units, about 40 scale units, about 50 scale units, about 60 scale units, about 70 scale units, about 80 scale units, about 90 scale units or about 100 scale units, measured at 25°C.

In this disclosure, where viscosity is indicated in scale units or scale divisions, it is measured on a Brookfield RVT DV-1 apparatus equipped with Helipath spindle C, rotating at 50 rotations per minute (rpm) at a temperature of 25°C, and the scale is read after about 2 minutes of rotation. Where viscosity is indicated in Pa.s, it is measured on a rotational viscosimeter such as MCR 150, MCR51 or equivalent cone/ plate viscosimeter, equipped with a cone type cone CP 50-1, rotating at about 100 rad/s at a temperature of 20°C, and the scale is read after about 2 minutes of rotation.

The compositions can further comprise a pH adjusting agent. The pH adjusting agents can be basic agents. Especially in embodiments comprising a carbomer gelling agent, the compositions comprise a basic agent. The basic agent can initiate the gelling of the carbomer gelling agent, which is a polyacrylic acid. The basic agent can be present in a concentration sufficient to adjust the pH to about 7 to about 8.5, preferably about 7.5 to about 8.2. pH measurements are usually carried out at 20°C. The basic agent can be any basic agent not inconsistent with the objectives of this disclosure. For example aliphatic amines, e.g. primary, secondary, and tertiary alkanolamines and primary, secondary, and tertiary alkylamines can be used. Specific examples include monoethanolamine, diethanolamine, diisopropanolamine, triethanolamine, triisopropanolamine, dimethylamine, diethylamine, trimethylamine, triethylamine. Preferred is diethylamine. Preferably, the API is diclofenac diethylammonium and the basic agent is diethylamine, because diethylamine as basic agent comprises the diethylammonium cation, which is the counter ion of the diclofenac diethylammonium salt.

Alternatively, the basic agent can be an inorganic, such as NaOH or ammonia. The basic agent can be provided in aqueous solution. In one embodiment, the basic agent is ammonia (conc. 10-30% w/v in water). Use of an inorganic base could reduce risk of nitrosamine formation, a storage risk for topical diclofenac products. In embodiments comprising carbomer gelling agents, ammonia solution is preferred over NaOH, as carbomer gelling agents are sensitive to ions, and the sodium ions of NaOH can reduce the carbomer gelling agent's gelling capacity, such that more carbomer gelling agents need to be added to achieve a certain viscosity. Moreover, ammonia can aid compounding clear transparent gels, whilst in some instances, Na-ions can render the compositions opalescent.

The compositions also comprise water. The water can be purified water, distilled water, or similar. The water can be pharma grade water. At least about 70% w/w of the compositions can be water. The compositions can comprise from about 70% w/w to about 85% w/w water, preferably about 75% w/w to about 82% w/w water. In one embodiment, the compositions comprise about 79% w/w water.

The compositions can typically have a pH from about 7 to about 8.5, preferably about 7.5 to about 8.2. Optionally, pH adjusting agents can be used to achieve this pH range. For example, buffers may be used, such as a phosphate buffer. In one embodiment, the pH of the composition is higher than 7.2. pH measurements are usually carried out at 20°C.

The compositions can optionally comprise further excipients, such as perfumes, chelating agents, preservatives, antioxidants, colorants, or the like.

Examples of perfumes are eucalyptus perfume, rose perfume, lavender perfume, rosemary perfume wintergreen perfume and mint perfume.

Examples of chelating agents are salts of edetic acid, malic acid, fumaric acid and citric acid.

Examples of preservatives are benzalkonium chloride, benzethonium chloride, benzoic acid, benzyl alcohol and sodium metabisulfite.

Examples of antioxidants are alpha tocopherol and butylated hydroxytoluene.

Examples of colorants are FD&C Blue #1, D&C Orange #5 and D&C Red #6.

In one embodiment, there is provided a composition comprising:
a) a pharmaceutically acceptable salt of an arylacetic acid nonsteroidal anti-inflammatory analgesic selected from alclofenac, diclofenac and felbinac,
b) a C₂-C₄-monoalcohol, in a concentration of about 12% w/w to about 15% w/w,
c) a C₂-C₄-polyalcohol, in a concentration of about 2.5% w/w to about 7.5% w/w,
d) a carbomer gelling agent,
   and water.

In another embodiment, there is provided a monophasic, hydroalcoholic gel for topical drug delivery comprising:
a) diclofenac sodium or diclofenac diethylammonium salt, in a concentration of about 0.5% w/w to about 1.5% w/w diclofenac sodium equivalent,
b) a C₂-C₄-monoalcohol, in a concentration of about 12% w/w to about 15% w/w,
c) a C₂-C₄-polyalcohol, in a concentration of about 2.5% w/w to about 7.5% w/w,
d) a carbomer gelling agent,
e) a basic agent to adjust the pH of the total composition to a pH of about 7.5 to about 8.5, and
f) water.

In another embodiment, there is provided monophasic, hydroalcoholic gel for topical drug delivery, comprising:
- diclofenac diethylammonium, in a concentration of about 1.16% w/w,
- isopropanol, in a concentration of about 12% w/w to about 15% w/w,
- propylene glycol, in a concentration of about 2.5% w/w to about 7.5% w/w,
- a carbomer gelling agent, in a concentration of about 0.7% w/w to about 1.5% w/w,
- a basic agent to adjust the pH of the total composition to a pH of about 7.5 to about 8.5, and
- water, in a concentration of about 70% w/w to about 85% w/w.

In another embodiment, there is provided a composition consisting essentially of:
a) diclofenac diethylammonium, in a concentration of about 1.16% w/w,
b) isopropanol, in a concentration of about 12% w/w to about 15% w/w,
c) propylene glycol, in a concentration of about 2.5% w/w to about 7.5% w/w,
d) a carbomer gelling agent, in a concentration of about 0.7% w/w to about 1.5% w/w,
e) diethylamine to adjust the pH of the total composition to a pH of about 7.5 to about 8.5, and
f) water, in a concentration of about 70% w/w to about 85% w/w,
   and optionally, perfumes, pH adjusting agents, gelling agents, emollients, humectants, preservatives, chelating agents, antioxidants and/or colorants.

In another embodiment, there is provided a composition consisting essentially of:
a) diclofenac diethylammonium, in a concentration of about 1.16% w/w,
b) isopropanol, in a concentration of about 12.5 w/w,
c) propylene glycol, in a concentration of about 5% w/w,
d) carbomer, in a concentration of about 0.7% w/w to about 1.5% w/w,
e) diethylamine to adjust the pH of the total composition to a pH of about 7.3 to about 8.2, and
f) water, in a concentration of about 70% w/w to about 85% w/w,
   and optionally, perfumes, pH adjusting agents, gelling agents, emollients, humectants, preservatives, chelating agents, antioxidants and/or colorants.

In another embodiment, there is provided a composition consisting essentially of:
a) diclofenac diethylammonium, in a concentration of about 1.16% w/w,
b) isopropanol, in a concentration of about 12.5 w/w,
c) propylene glycol, in a concentration of about 5% w/w,
d) carbomer, in a concentration of about 1.2 % w/w,
e) diethylamine in a concentration of about 1 % w/w,
f) perfume, in a concentration of about 0.1 % w/w, and
g) water, ad. 100%.

Preferably, the compositions disclosed herein are substantially free from lipophilic excipients. Preferably, the compositions do not contain a lipophilic or fatty phase. This is to provide the consumers a "fat-free", "no-messy", "non-greasy" alternative, completely based on water and alcohols. Multi-phase systems comprising a lipophilic phase are typically opaque, milky or cloudy. However, by generally excluding lipophilic excipients and a lipophilic phase, it is possible to obtain a clear, transparent composition. However, excluding such lipophilic excipients and liophilic phases is challenging because lipophilic excipients and phases are often used as permeation enhancers, solubilizers or skin conditioning agents in topical formulations. Therefore it is challenging to successfully formulate a hydrophilic, monophasic composition without lipophilic ingredients that provides sufficient long term solubilzation and permeation of the API, and still has desireable sensory attributes, e.g. ease of application, hydrating properties and the like. Specifically, application characteristics of Voltaren 1.16% Emulgel that are perceived as positive by users, should be retained. Preferably, the compositions disclosed herein are substantially free from permeation enhancers. Typically, permeation enhancers are included in compositions to increase cumulative permeation and/or flux of the API. However, they can be irritating to skin. Additionally, some permeation enhancers need to be specifically highlighted on package labels. Specifically, the compositions can be free from permeation enhancers like isostearic acid and other fatty acids or acids. Permeation enhancers can yield cloudy, hazy or milky compositions. Specifically, the compositions are preferably free from fatty alcohols, fatty acids, isopropylmyristate, isopropyl palmitate and diethyl sebacate. As explained above, the present inventors aimed at formulating a composition with permeation characteristics similar to those of the existing Voltaren 1% or 1.16% emulgel products, which surprisingly could be achieved without incorporation of permeation enhancers.

Preferably, the compositions disclosed herein are substantially free from emulsifying agents. Emulsifying agents often can yield cloudy, hazy or milky compositions and therefore are not preferred.

Preferably, the compositions disclosed herein are substantially free from humectants. Whilst humectants are often included in topical compositions for their cooling, conditioning and hydrating properties, they can lead to long drying time of a product on the skin. Therefore they are not preferred.

Preferably, the compositions disclosed herein are substantially free from sensates. For example, the compositions can be free from peppermint oil, 1-menthol and menthol derivatives, methyl salicylate, ethyl salicylate, glycol monosalicylate or the like. Preferably, the compositions are substantially free from counter irritants. Preferably, the compositions are substantially free from inductive stimulants. Sensates, counter irritants and inductive stimulants are often incorporated to provide the "fast acting" or cooling sensation that was described above as a preferred senosory attribute. However, they can also cause turbidity or phase separation in formulations. They can also lead to irritiation. Furthermore, they can influence permeation of the compositions.

Attributes of the disclosed compositions

The compositions preferably are transparent gels. Preferably, the compositions are colourless and transparent. In some embodiments, the compositions can be slightly opalescent.

As discussed above, it is important that the API is fully solubilized. This can be determined by the absence of API crystals. Preferably the compositions are free from crystals of API upon visual inspection with a light microscope. Preferably, the compositions are homogenous upon visual inspection with the bare eye. Preferably, the compositions are free from crystals of the API over a shelf life of 24 months.

In one embodiment, the compositions are free from crystals upon inspection with a microscope with 40X optical zoom. In one embodiment, the compositions are free from crystals upon inspection with a microscope with 40X optical zoom, after storage for 3 months at 25°C, 60% relative humidity (RH). In one embodiment, the compositions are free from crystals upon inspection with a microscope with 40X optical zoom, after storage for 3 months at 40°C, 75% RH.

Preferably, the compositions are homogenous upon visual inspection with a light microscope.

The compositions of this disclosure can have a drying time of less than 120 seconds, preferably less than 110 seconds. In one embodiment, the compositions of the invention have a drying time of less than 100 seconds. In some embodiments, the composition have a drying time of 40 seconds or less, 50 seconds or less, 60 seconds or less, 70 seconds or less, 80 seconds or less, 90 seconds or less, 100 seconds or less, 110 seconds or less or 120 seconds or less. The method of measuring the drying time is explained in detail in the example section.

As explained above, the compositions of this disclosure provide the desired sensory attributes. For example, the compositions are easy to spread on skin. The compositions also provide a cooling sensation upon rubbing in, which even persists after rubbing in. The compositions do not leave residues or flakes or a film on the skin after drying.

### Cumulative skin permeation

In one embodiment, the compositions have a cumulative permeation in an in vitro skin permeation test that is equal to or greater than the cumulative permeation of Voltaren 1.16% Diclofenac Diethylammonium emulgel.

In one embodiment, the compositions have a cumulative permeation in an in vitro skin permeation test, that enables to bridge the literature data of Voltaren 1.16% Diclofenac Diethylammonium emulgel, in view of well-established use registration, according to Annex I of Directive 2001/83/EC. "Similar permeation characteristics" in the present disclosure means, that the permeation profile of a formulation is such that in vitro skin permeation test results, comparing the skin permeation of the formulation to Voltaren 1.16% Diclofenac Diethylammonium emulgel, can be used to bridge the literature data of Voltaren 1.16% Diclofenac Diethylammonium emulgel, in view of well-established use registration, according to Annex I of Directive 2001/83/EC.

### METHODS OF MANUFACTURE

The compositions can be manufactured as follows:
- Combining the complete amounts of C₂-C₄-monoalcohol, C₂-C₄-polyalcohol, API, and a portion of the water (about 3% to about 4% of the total of water),
- Mixing this combination until the API is completely dissolved to obtain an API phase;
- Optionally adding, under mixing, additional, optional ingredients to the API phase;
- Optionally, diluting the basic agent in about 1.5% of the total amount of water;
- Dispersing the carbomer gelling agent in the remaining amount of water, until it is fully dispersed, to obtain a carbomer-water dispersion;
- Adding the (optionally diluted) basic agent to the carbomer-water dispersion to initiate gelling of the carbomer to obtain an aqueous carbomer gel;
- Combining the aqueous carbomer gel and the API phase under homogenization.

### DEFINITIONS

Unless indicated otherwise, concentrations are given in % are weight% (w/w).

Where the term "about" is applied to a particular value (e.g. "about 200°C") or to a range (e.g. "about x to about y"), the value or range is to be interpreted as being as accurate as the method used in the art to measure it.

If not indicated otherwise, pH is measured at 20°C.

### USE

The compositions disclosed herein are suitable for use in the treatment of joint pain, osteoarthritis, muscle pain, back pain and/or inflammation.

### PHARMACEUTICAL COMPOSITIONS/ROUTES OF ADMINISTRATION/DOSAGES

Compositions described herein can be useful in the treatment of joint pain, osteoarthritis pain, back pain, knee pain, ankle pain, neck pain, muscle pain, sprains, and/or inflammation. The compositions can be administered to mammals suffering from these ailments. Preferably, the compositions are for the treatment of humans.

They can be applied onto the skin covering the concerned body part. They may be rubbed in until the skin feels dry. They may be applied 2-4 times per day. In some embodiments, a dose of the compositions can comprise up to 2grams per application for the upper extremities, and up to 4grams per application for the lower extremities at a 2-4 times per day application. The dose can thus be up to 80mg diclofenac sodium equivalent per day for the upper extremities and up to 160mg diclofenac sodium equivalent per day for the lower extremities in many cases. The methods of treatment can comprise administering to a subject in need thereof a pharmaceutically effective amount of the compositions disclosed herein. The method can comprise administering the composition to skin that covers a body part suffering from or causing one or more of joint pain, osteoarthritis, muscle pain, back pain and/or inflammation.

Further disclosed herein are methods of treatment of joint pain, osteoarthritis pain, back pain, knee pain, ankle pain, neck pain, muscle pain, sprains, and/or inflammation, the methods comprising:
g) Applying to the skin of a site of application of a patient up to approximately 2 grams on upper extremities or up to approximately 4grams on lower extremities of a gel according to any of the compositions described herein,
h) Rubbing or massaging the gel into the skin until the application site is dry,
i) Repeating the steps a) and b) 3 to 4 times in 24 hours, until treatment is ceased.

The compositions can be provided in a package comprising a means for dosing. The compositions may be provided in a container comprising an applicator for direct application onto the skin. Embodiments described herein can be understood more readily by reference to the following detailed description, examples, and figure. Elements, apparatus, and methods described herein, however, are not limited to the specific embodiments presented in the detailed description, examples, and figures. It should be recognized that the exemplary embodiments herein are merely illustrative of the principles of the present invention. Numerous modifications and adaptations will be readily apparent to those of skill in the art without departing from the spirit and scope of the invention.

In addition, all ranges disclosed herein are to be understood to encompass any and all subranges subsumed therein. For example, a stated range of "1.0 to 10.0" should be considered to include any and all subranges beginning with a minimum value of 1.0 or more and ending with a maximum value of 10.0 or less, e.g., 1.0 to 5.3, or 4.7 to 10.0, or 3.6 to 7.9.

All ranges disclosed herein are also to be considered to include the end points of the range, unless expressly stated otherwise. For example, a range of "between 5 and 10" or "5 to 10" or "5-10" should generally be considered to include the end points 5 and 10.

It is further to be understood that the feature or features of one embodiment may generally be applied to other embodiments, even though not specifically described, or illustrated in such other embodiments, unless expressly prohibited by this disclosure or the nature of the relevant embodiments. Likewise, compositions and methods described herein can include any combination of features and/or steps described herein not inconsistent with the objectives of the present disclosure. Numerous modifications and/or adaptations of the compositions and methods described herein will be readily apparent to those skilled in the art without departing from the present subject matter.

### EXAMPLES

Hereinafter, the present invention will be described in more detail with reference to Examples, but the present invention is not limited by these Examples.

### Example 1: Formulation experiments

To formulate a topical product that addresses the challenges discussed above, the present inventors have conducted a formulation experiment. Formulations with different composition were produced, see Table 1 below. pH and apparent viscosity were measured, and visual appearance was analysed with the bare eye. Apparent viscosity was measured on a Brookfield RVT DV-1 apparatus equipped with Helipath spindle C, rotating at 50 rotations per minute (rpm) at a temperature of 25°C, and the scale was read after about 2 minutes of rotation. The values are indicated in Table 1 for the apparent viscosity is indicated in scale units according to this measurement method. The results are also given in Table 1.

**Table 1: Composition and characteristics of formulations of Example 1**

| Ingredient | Form ulati on 1 | Formul ation 2 | Formul ation 3 | Formul ation 4 | Formul ation 5 | Formul ation 6 | Formul ation 7 | Formul ation 8 | Formul ation 9 | Formul ation 10 | Formul ation 11 | Formul ation 12 | Formul ation 13 | Formul ation 14 | Formul ation 15 | Formul ation 16 | Formul ation 17 |
|---|---|---|---|---|---|---|---|---|---|---|---|---|---|---|---|---|---|
| Diclofenac Diethylami ne | 1.16 | 1.16 | / | 1.16 | 1.16 | 1.16 | 1.16 | 1.16 | 1.16 | 1.16 | 1.16 | 1.16 | 1.16 | 1.16 | 1.16 | 1.16 | 1.16 |
| Carbomer 974P | 1.20 | 1.20 | 0.80 | 1.20 | 1.20 | 1.20 | 1.20 | 1.20 | 1.20 | 1.20 | 1.20 | 1.20 | / | / | / | / | 1.20 |
| Carbomer 980 | / | / | / | / | / | / | / | / | / | / | / | / | 1.80 | 1.60 | 1.60 | 1.20 | / |
| Isopropyl alcohol | 20.0 0 | 7.50 | 12.50 | 10.00 | 5.00 | 5.00 | 12.50 | 10.00 | 12.50 | 12.50 | 11.00 | 8.00 | 12.50 | 12.50 | 12.50 | 12.50 | 4.50 |
| Ethanol 98% | / | / | / | / | / | / | / | / | / | / | / | 4.50 | / | / | / | / | 8.00 |
| Propylene glycol | 5.00 | 12.50 | 12.50 | 5.00 | 5.00 | 3.50 | 5.00 | 7.50 | 7.50 | 5.00 | 5.00 | 5.00 | 5.00 | 5.00 | 5.00 | 5.00 | 5.00 |
| Diethylami ne | 0.90 | 0.90 | 0.60 | 0.90 | 0.90 | 0.90 | 0.90 | 0.90 | 0.90 | 0.94 | 0.90 | 0.90 | 1.42 | 1.42 | 1.35 | 1.01 | 0.90 |
| Perfume cream 45 | 0.10 | 0.10 | / | 0.10 | 0.10 | 0.10 | 0.10 | 0.10 | 0.10 | 0.10 | 0.10 | 0.10 | 0.10 | 0.10 | 0.10 | 0.10 | 0.10 |
| Purified Water | 71.6 4 | 76.64 | 73.60 | 81.64 | 86.64 | 88.14 | 79.14 | 79.14 | 76.64 | 79.10 | 80.64 | 79.14 | 78.02 | 78.22 | 78.29 | 79.03 | 79.14 |
| Results | | | | | | | | | | | | | | | | | |
| pH | 7.27 | 7.35 | 7.71 | 7.25 | 7.49 | 7.52 | 7.38 | 7.38 | 7.32 | 7.57 | 7.25 | 7.47 | 7.36 | 8.24 | 7.76 | 7.71 | 7.35 |
| Apparent viscosity | 58.0 0 | 50.50 | 83.3 | 56.3 | 78.1 | 75.5 | 56 | 62.2 | 57.8 | 62 | 55.1 | 57.8 | 90 | 88.4 | >100 | 57.2 | 61.7 |

| Visual appearance | | | | | | | | | | | | | | | | | |
|---|---|---|---|---|---|---|---|---|---|---|---|---|---|---|---|---|---|
| Colour | 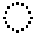 | 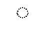 | 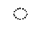 | 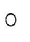 | • | • | 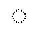 | 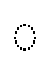 | 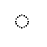 | 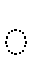 | 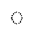 | 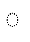 | 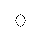 | 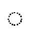 | 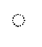 | 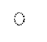 | 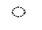 |
| Homogenei ty on day 0 | ◇ | ◇ | ◇ | ◇ | ◆**◆** | **◆◆** | ◇ | ◇ | ◇ | ◇ | ◆, 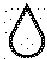 | ◇ | ◇ | ◇ | ◇ | n.a. | n.a.◇ |
| Homogenei ty on day 1 | ◇ | ◇ | ◇ | **◆◆** | **◆◆** | **◆◆** | ◇ | ◇ | ◇ | ◇ | ◆, 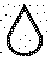 | ◇ | ◇ | n.a. | n.a. | n.a. | n.a. |
| Homogenei ty on day 3 | ◇ | ◇ | ◇ | **◆◆** | **◆◆** | **◆◆** | ◇ | **◆◆** | **◆◆** | n.a. | n.a. | ◇ | n.a. | n.a. | n.a. | n.a. | n.a. |

| | | | | | | | | | | | | | | | | | |
|---|---|---|---|---|---|---|---|---|---|---|---|---|---|---|---|---|---|
| Legend: ◇ no crystals present, homogenic ◆ crystals present **◆◆** many crystals present 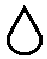 droplets visible with the bare eye 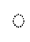 clear, colourless to slightly opalescent ∘ greyish colour or hazy appearance • whitish to white colour n.a. not tested | | | | | | | | | | | | | | | | | |

### Example 2: In vitro skin permeation study

An in vitro skin permeation study was conducted in a preclinical laboratory. Skin permeation studies can be used to determine the qualitative permeation characteristics of a formulation through, e.g., human skin. They can also be used for direct quantitative comparison the permeation and flux of two given formulations. It was the objective of this experiment to determine if any of the screening formulations has a permeation profile similar to the reference product. Five of the screening formulations of Example 1, namely Formulation 1, 2, 4, 7 and 9, were applied to human donor abdominal skin at timepoint 0h in a dose of 10mg/cm². Each formulation was tested on five skin samples from different donors on a Franz diffusion cell. The reference product for comparison was Voltaren Emulgel 1.16% (m/m) Diclofenac diethylammonium. The composition of Voltaren Emulgel 1.16% (m/m) is given in Table 2. The concentration of diclofenac in the receptor fluid of the Franz cell was measured at timepoints t=0, 4h, 8h and 24h, and the study endpoint was the cumulative amount of diclofenac permeated at 24h. The results of the study are given in Table 3.

**Table 2: Composition of Voltaren Emulgel 1.16% (w/w)**

| Ingredient | Concentration [% w/w] |
|---|---|
| Diclofenac Diethylammonium | 1.16 |
| Isopropyl alcohol | 20.00 |
| Propylene glycol | 5.00 |
| Cocoyl caprylocaprate | 2.50 |
| Paraffin, liquid | 2.50 |
| Cetomacrogol 1000 | 2.00 |
| Carbopol 974P | 1.20 |
| Diethylamine | 0.90 |
| Perfume cream | 0.10 |
| Water, purified | 64.64 |

**Table 3: Results of the in vitro skin permeation study of Example 2**

| | Reference product | Formulation 1 | Formulation 2 | Formulation 4 | Formulation 7 | Formulation 9 |
|---|---|---|---|---|---|---|
| N | 5 | 5 | 4 | 5 | 5 | 5 |
| Min | 1632 | 2071 | 2929 | 3591 | 2825 | 4923 |
| Median | 4098 | 9101 | 5577 | 6941 | 3869 | 8300 |
| Max | 5176 | 27165 | 20689 | 17993 | 28338 | 20828 |
| Geometric mean | 3419 | 7900 | 6589 | 8678 | 5757 | 9445 |
| CV (%) | 49.11 | 148.14 | 98.10 | 76.46 | 122.45 | 66.16 |
| Ratio vs. reference product [90% CI] | n.a. | 2.31 [0.96; 5.58] | 2.08 [1.21; 3.58] | 2.54 [1.85; 3.47] | 1.68 [0.82; 3.45] | 2.76 [1.87; 4.09] |

It was found that Formulation 7 had a cumulative permeation closest to the reference product.

### Example 3: Sensory panel testing

To test the sensory attributes of the compositions, Formulation 7, in a placebo version, for security reasons, was compared to Voltaren 1.16% Diclofenac Diethylammonium Emulgel in a sensory panel experiment. Eleven subjects participated in the study. Both products were assessed each day with a 20-minute break between. Two replicates of product assessments were conducted. Each product was assessed twice under different blind codes on separate days. Each panellist had a separate rotation plan and the leg used was randomised across the replications. All but one of the panel were right-handed. The preparation protocol was as follows: Carefully weigh out 2g of the sample onto a plastic spoon. Serve spoon with 2g weighed sample to the panellist. The application protocol was as follows: Wash hands and dry thoroughly. Wipe back of leg (calf) with Huggies unperfumed wet wipe and allow to dry. Panellist scoops the whole contents of the spoon onto fingertips. Use the left hand for the left calf, right hand for right calf. Apply product to the calf, set timer and rub the sample into the skin with circular motions using the whole hand until dry. Once dry, stop the timer and note the length of time taken. Repeat for second sample using the other leg and other hand.

This is also the protocol with which "drying time" as discussed above, was measured.

In addition to the drying time, the following attributes were assessed: Ease of absorption (overall during rubbing in), ease of spreading (overall during rubbing in), stickiness (could be assessed during and at end), cooling feel (during and at end), tight feel on skin (immediately after rubbing in) and residue (immediately after rubbing in).

The results are reproduced in Table 4 below.

**Table 4: Results of Sensory panel testing**

| Attribute | Current Voltaren 1% | | Formulation 7 placebo version | |
|---|---|---|---|---|
| Time to rub in until dry (secs) | 70.9 | b | 99.0 | a |
| Ease of absorption while rubbing in | 61.4 | a | 43.5 | b |
| Ease of spreading while rubbing in | 72.7 | b | 78.0 | a |
| Stickiness while rubbing in | 39.0 | a | 35.8 | a |
| Cooling while rubbing in | 33.0 | b | 44.0 | a |
| Tight feel on skin immediately after rubbing in | 22.5 | a | 29.0 | a |
| Residue immediately after rubbing in | 30.4 | b | 34.8 | a |
| Stickiness after rubbing in | 38.1 | b | 47.8 | a |
| Cools after rubbing in | 27.5 | b | 40.1 | a |

| | | | | |
|---|---|---|---|---|
| Note: a-b indicates significant difference at 95% confidence. | | | | |

The same alphabet letter indicates no significant differences at 95% confidence limit.

Different alphabet letters indicate a significant difference in sensory scores at the 95% confidence limit.

Formulation 7 thus dries in less than 100 seconds, which is comparable to the reference product. This is surprising, because Formulation 7 contains less isopropanol, i.e. less volatile solvent. Significantly, Formulation 7 is not stickier than the reference product. Formulation 7 also does not leave a tighter feel on the skin. Formulation 7 significantly easier to spread. This is surprising, as it does not contain a fatty phase. Importantly, Formulation 7 feels significantly cooler both while rubbing in and after rubbing in.

### Example 4: Visual appearance

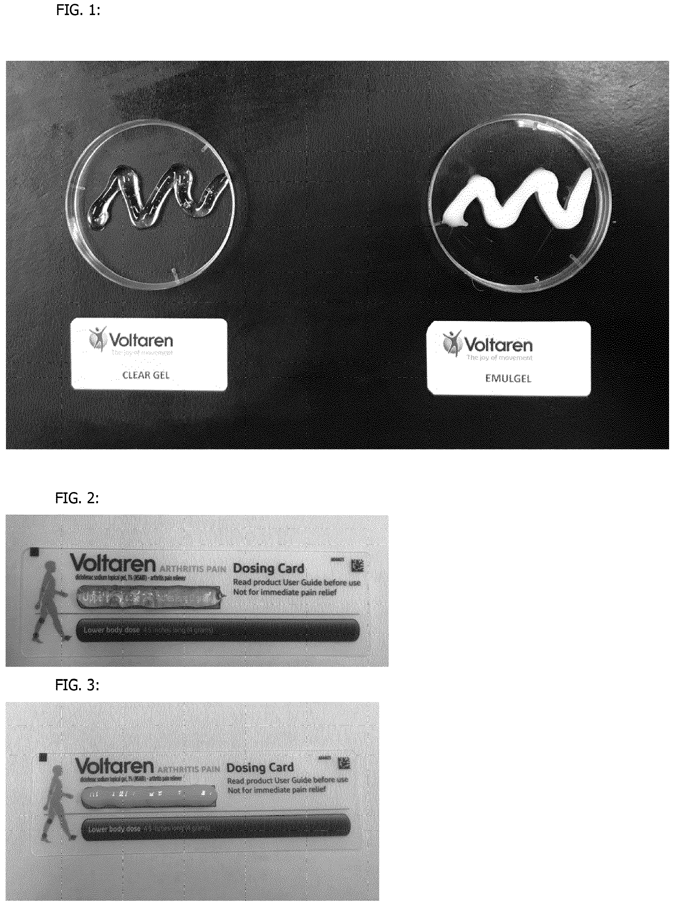

As discussed above, the gels disclosed herein have an excellent visual appearance. FIG. 1 is a representative photograph illustrating the visual appearance of a composition of this disclosure (on the left, labelled as "CLEAR GEL") in comparison to Voltaren 1.16% Emulgel (on the right, labelled as "EMULGEL"). FIG. 2 is a representative photograph of a composition of this disclosure on a typical dosing card for topical products. FIG. 3 is a representative photograph of Voltaren 1.16% Emulgel on a typical dosing card for topical products. These dosing cards are in many cases used to measure a specific dose of a topical product. A dosing card usually is a plastic card comprising a printed line of a predefined length. The printed line is used as a reference. For example, a dosing card can comprise a printed line of a length of 6cm, which corresponds to a predefined dose. A user can position the dosing card on his or her skin, and then apply a line of product, having the same length as the reference line on the dosing card, onto the skin. Alternatively, a user can dose a line of product onto the reference line on the dosing card, and then apply the product from the dosing card to the desired skin area. The embodiment of the disclosure can be described as clear, crystal clear, transparent or see-through. As apparent from FIG. 2, it is even possible to discern the text on the dosage card on the reference line underneath the line of gel. By contrast, as is apparent from FIG. 3, this is not the case for Voltaren 1.16% Emulgel. Voltaren 1.16% Emulgel appears white opaque, so that the underlying reference line and text are not visible.

## Claims

1. A monophasic, hydroalcoholic gel for topical drug delivery, comprising g) diclofenac epolamine or diclofenac diethylammonium, in a concentration of about 0.5% to about 1.5% w/w,
h) a C₂-C₄-monoalcohol, in a concentration of about 12% to about 15% w/w,
i) a C₂-C₄-polyalcohol, in a concentration of about 2.5% to about 7.5% w/w,
j) a gelling agent,
k) optionally, pH adjusting agent,
l) water,
wherein the pH of the total composition is from about 6.5 to about 8.5.

2. A gel according to claim 1, wherein b) is isopropanol.

3. A gel according to claim 1 or 2, wherein c) is propylene glycol.

4. A gel according to any of claims 1, 2 or 3, wherein b) and c) together are present in a concentration of about 15% to about 25% w/w.

5. A gel according to claim 4, wherein b) and c) together are present in a concentration of about 15% to about 20% w/w.

6. A gel according to any of the preceding claims, wherein the ratio of b) to c) is from about 1:1 to about 5:2.

7. A gel according to any of the preceding claims, wherein b) is present in a concentration of about 12% to about 13% w/w.

8. A gel according to any of the preceding claims, wherein c) is present in a concentration of about 4% to about 6% w/w.

9. A gel according to any of the preceding claims, wherein d) is a Carbomer gelling agent.

10. A gel according to any of the preceding claims, wherein e) is a basic agent.

11. A gel according to any of the preceding claims, wherein a) is diclofenac diethylammonium.

12. A gel according to any of the preceding claims, wherein f) is present in a concentration of more than about 70% w/w.

13. A gel according to any of the preceding claims, wherein d) is present in a concentration of about 0.7% to about 1.5% w/w.

14. A gel according to any of the preceding claims, wherein the gel is substantially free from permeation enhancers.

15. A gel according to any of the preceding claims, wherein the gel is substantially free from emulsifying agents.

16. A gel according to any of the preceding claims, wherein the gel is substantially free from humectants.

17. A gel according to any of the preceding claims, wherein the gel does not contain a lipophilic phase.

18. A gel according to any of the preceding claims, wherein the viscosity of the gel is from about 1Pa.S to about 5Pa.S.

19. A gel according to any of the preceding claims, wherein the gel has a drying time on skin of less than 120 seconds.

20. A gel according to any of the preceding claims, having a cumulative permeation in an in vitro skin permeation test, that enables to bridge the literature data of Voltaren 1.16% Diclofenac Diethylammonium emulgel, in view of well-established use registration, according to Annex I of Directive 2001/83/EC.

21. A gel according to any of the preceding claims, wherein the gel is transparent.

22. A gel according to any of the preceding claims, consisting essentially of
a) diclofenac diethylammonium, in a concentration of about 1% to about 1.2% w/w,
b) isopropanol, in a concentration of about 12% to about 13% w/w,
c) propylene glycol, in a concentration of about 4% to about 6% w/w,
d) Carbomer, in a concentration of about 0.7% to about 1.5% w/w,
e) diethylamine, in a concentration of about 0.7% to about 1.3% w/w,
f) optionally, one or more of perfumes, pH adjusting agents, gelling agents, emollients, humectants, preservatives, chelating agents, antioxidants and/or colorants.

23. A gel according to any of the preceding claims, provided in an applicator pack for direct application to the skin.

24. A gel according to any of the preceding claims, for use in a method of providing a cooling sensation on mammal skin on an application site.

25. A gel according to any of the preceding claims, for use in the treatment of a human suffering from joint pain, osteoarthritis pain, back pain, knee pain, ankle pain, neck pain, muscle pain, sprains, and/or inflammation.

26. A gel for use according to claim 28 or 29, wherein the gel is applied to the skin of a site of application in a dose of 2grams on upper extremities or 4 grams on lower extremities, the gel is rubbed or massaged into the skin until the application site is dry, and the application is repeated 3 to 4 times in 24 hours, until treatment is ceased.
